# EUROPEAN PATENT SPECIFICATION

(11) **EP 0 813 517 B1**
(45) Date of publication and mention of the grant of the patent: **14.11.2001**
(21) Application number: 96904623.4
(22) Date of filing: 09.02.1996
(51) Int. Cl.: C07C 209/72, C07C 5/10

(54) **HYDROGENATION OF UNSATURATED CYCLIC COMPOUNDS**
HYDROGENIERUNG UNGESÄTTIGTER VERBINDUNGEN
HYDROGENATION DE COMPOSES CYCLIQUES INSATURES

(30) Priority: 06.03.1995 US 398690; 14.03.1995 US 404095
(43) Date of publication of application: 29.12.1997
(73) Proprietor: CHEMICAL RESEARCH & LICENSING COMPANY, Pasadena, Texas 77507 (US)
(72) Inventor: GILDERT, Gary, R., Pasadena, TX 77507 (US); HEARN, Dennis, Pasadena, TX 77507 (US); PUTMAN, Hugh, M., Pasadena, TX 77507 (US); NEMPHOS, Speros, Peter, Pasadena, TX 77507 (US)
(74) Representative: Baillie, Iain Cameron
(86) International application number: US9601950
(87) International publication number: WO9627580

(56) References cited:
- US-A- 4 914 239

## Description

The present invention relates to the hydrogenation of unsaturated cyclic and polycyclic compounds. More particularly the invention relates to a process wherein the hydrogenation of the unsaturated cyclic and polycyclic compounds and separation of the product by distillation is carried out simultaneously in a distillation column reactor. More particularly the process relates to the hydrogenation of benzene to make cyclohexane and to a process for the production of cyclohexyl amine by hydrogenation of aniline.

### Related Information

Cyclohexane is the main precursor for the production of nylon products and as such the demand remains strong. Cyclohexane was first obtained by the direct fractional distillation of suitable crude petroleum refinery streams. Now the major portion of cyclohexane is obtained from the direct hydrogenation of benzene.

Peterson in U.S. Pat. No. 2,373,501 discloses a liquid phase process for the hydrogenation of benzene to cyclohexane wherein a temperature differential is maintained between the top of the catalyst bed where benzene is fed and the outlet where substantially pure cyclohexane is withdrawn. The temperature differential is due to the change in the exothermic heat of reaction released as less and less benzene is converted as the concentration of benzene decreases. Hydrogen is supplied counter current to the benzene/cyclohexane flow. Temperature control coils are disposed within the reactor to maintain the temperature differential if the exothermic heat of reaction is not sufficient or to cool the bed if too much heat is released. Peterson recognizes that although the bulk of his reaction takes place in the liquid phase a portion of the benzene and cyclohexane will be vaporized, especially near the top of the reactor where the benzene concentration is highest and conversion is highest. A reflux condenser is provided to condense the condensible material and return it to the reactor. Thus a substantial portion of the heat of reaction is removed by condensation of the reactants vaporized throughout the reaction.

Larkin, et al. in U.S. Pat. No. 5,189,233 disclose another liquid phase process for the hydrogenation of benzene to cyclohexane. However, Larkin, et al utilize high pressure (2500 psig) to maintain the reactants in the liquid state. In addition Larkin, et al disclose the use of progressively more active catalyst as the concentration of benzene decreases to control the temperature and unwanted side reactions.

Hui, et al. in U.S. Pat. No. 4,731,496 disclose a gas phase process for the hydrogenation of benzene to cyclohexane over a specific catalyst. The catalyst reported therein is nickel supported on a mixture of titanium dioxide and zirconium dioxide.

The hydrogenation of benzene is also useful to remove that aromatic compound from gasoline streams. One example of this process is disclosed by Hsieh, et al in U.S. Pat. No. 5,210,348 wherein hydrogenation of the benzene fraction is used alone or in combination with alkylation. In some schemes for the reduction of aromatic compounds in gasoline the ASTM D-86 90% point is specified such that the aromatic and unsaturated cyclic and polycyclic compounds are precluded from the gasoline blending pool. This has been termed a T-90 gasoline stock having a desired ASTM 90% point. The resultant T-90+ bottoms which are largely unsaturated cyclic and polycyclic compounds must be disposed of and hydrogenating them to produce lighter more saturated compounds for the gasoline pool is an attractive alternative.

A typical problem with the hydrogenation of benzene to cyclohexane is the competing reactions. Particularly isomerization to methyl cyclopentane is unwanted. Additionally at higher temperatures cracking of the ring occurs producing undesirable C₅ and lighter products.

Cyclohexyl amine has been produced by a number of processes in the past including amination of cyclohexanol and the hydrogenation of aniline. The major difficulty encountered in the hydrogenation of aniline is the formation of varying amounts of dicyclohexyl amine byproduct.

US-A-4,914,239 discloses continuous catalytic production of a cyclohexylamine by liquid phase hydrogenation of an aniline in the presence of an ammonia compound, removing a product gas, cooling to obtain a liquid phase and.distilling the liquid to obtain cyclohexylamine.

Catalyst known to promote the hydrogenation of aniline are metals in Ground VIII of the periodic table. In the past the proclivity to produce dicyclohexyl amine is rated from the least likely to the most likely as Ruthenium < rhodium < palladium = platinum. Supports used for the metals are carbon, barium carbonate, alumina, barium sulfate and calcium carbonate. The supports also affect the production of dicyclohexyl amine in the order carbon > barium carbonate > alumina> barium sulfate > calcium carbonate.

According to the present invention there is provided a process for the hydrogenation of unsaturated cyclic compounds comprising the steps of:
(a) feeding a first stream containing unsaturated cyclic compounds and a second stream containing hydrogen to a distillation column reactor;
(b) contacting the unsaturated cyclic compounds and hydrogen at a temperature in the range of 37° to 190°C (100° to 374°F), a hydrogen partial pressure of less than 345 kPa (50 psia), and an overhead pressure in the range of 101 to 929 kPa (0 to 120 psig) in the presence of a bed of hydrogenation catalyst prepared in the form of a catalytic distillation structure thereby reacting a portion of the unsaturated cyclic compounds with a portion of the hydrogen to form a reaction mixture containing saturated cyclic compounds, unreacted hydrogen and unreacted unsaturated cyclic compounds;
(c) maintaining the pressure in the distillation column reactor (i) to contain a vapor phase and some liquid phase and provide a continual reflux such that the reaction mixture is at its boiling point and boiling in the bed of catalyst and (ii) condensing a portion of the vapors in the reaction system whereby a portion of the aromatics and other unsaturated cyclic and polycyclic compounds is always condensing on the catalyst structure;
(d) removing gaseous unsaturated cyclic compounds, gaseous saturated cyclic compounds and hydrogen as overheads from the distillation column reactor;
(e) condensing substantially all of the unsaturated cyclic compounds and saturated cyclic compounds removed as overheads from the distillation column reactor;
(f) returning a portion of the condensed unsaturated cyclic compounds and saturated cyclic compounds to the distillation column reactor as reflux; and
(g) withdrawing a liquid product containing saturated cyclic compounds and unreacted unsaturated cyclic compounds from the condensed overheads.

The present invention comprises feeding a hydrocarbon stream containing aromatics and other unsaturated cyclic and polycyclic compounds, particularly benzene, along with a hydrogen stream at an effectuating hydrogen partial pressure of less than 345 kPa (50 psia) preferably from 14 to 172 kPa (2 to 25 psia) to a distillation column reactor containing a hydrogenation catalyst which is a component of a distillation structure and hydrogenating a portion of the aromatics and other unsaturated cyclic and polycyclic compounds.

The present invention uses catalytic distillation in the hydrogenation of aniline to take advantage of the condensing distillate within the distillation reaction zone in the distillation column reactor. Actual separation may only be a secondary consideration. The operation of the distillation column reactor results in both a liquid and vapor phase within the distillation reaction zone. A considerable portion of the vapor is hydrogen and ammonia while a portion is vaporous aniline. Within the distillation reaction zone there is an internal reflux and liquid from an external reflux which cool the rising vaporous aniline condensing a portion within the bed.

### BRIEF DESCRIPTION OF THE DRAWINGS

FIG. 1 is a schematic flow diagram of one embodiment of the invention hydrogenating benzene.

FIG. 2 is flow diagram in schematic form of one embodiment of the present invention hydrogenating aniline.

### DETAILED DESCRIPTION OF THE INVENTION

To provide the desired degree of temperature and residence time control a process and apparatus is provided wherein the reaction liquid is boiling within a distillation column reactor. Overheads are withdrawn and condensed with some of the condensate being returned to the distillation column reactor as reflux. The advantage of the present process is that due to the continual reflux a portion of the aromatics and other unsaturated cyclic and polycyclic compounds is always condensing on the catalyst structure.

Without limiting the scope of the invention it is proposed that the mechanism that produces the effectiveness of the present process is the condensation of a portion of the vapors in the reaction system, which occludes sufficient hydrogen in the condensed liquid to obtain the requisite intimate contact between the hydrogen and the benzene in the presence of the catalyst to result in their hydrogenation. Additionally, the vaporization of the liquid feed removes a substantial amount of the exothermic heat of reaction. Since the liquid is at the boiling point in the reactor, the temperature may be controlled by the pressure. An increase in pressure increases the temperature and a decrease in pressure decreases the temperature.

The terms "cyclic" and "polycyclic" compounds used herein include organic compounds of 2-50 carbon atoms and oxygen, nitrogen sulfur and combinations thereof.

A preferred class comprising structure of the formula: 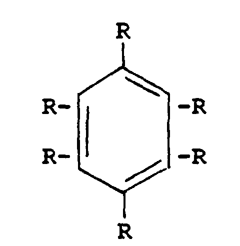 wherein each R is independently selected from hydrogen, alkyl, alkenyl, alkylaryl, NH₂, saturated and unsaturated cyclic structures formed by two R, containing C, O, N, S, or combinations thereof.

Some specific compounds include benzene, toluene, xylene, ethyl benzene, diethylbenzene, cumene, diisopylbenzene, phenol, durene, pentamethylbenzene, naphthalene, 1,2,3,4-tetrahydronapthalene, 1-methylnaphthalene, diphenylmethane, 2,2',3,3',4,4',5,5',6-nonomethyldiphenylmethane, hexamethylbenzene, 1,2,4,5,6,8-hexamethylanthracene, pentamethylphenol, durenol, mesitol, methyldiphenylmethane, pentamethylphenol, 1,1-binaphthyl, 1,2,3,4-tetrahydronaphthalene (tetralin), 4,4'-dimethyl-1,1'-binaphthyl, triphenylmethane, p-dibenzylbenzene, tetramethyldiphenylmethane, furan, thiophene, pyrrole, isopyrrole, pyrazole, 2-isoimidazole, 1,2,4-triazole, 1,2-dithiole, 1,2,3-oxyathiole, thiazole, 1,2-pyran, 1,4-pyrone, 1,2-dioxin, pyridine, aniline, triazive, 1,3,2-oxazine, 1,2,5-oxathiazine, azepine, indene, benzofurane, indole, benzoxazole, coumarin, quinayoline, phenanthrene, benzonaphthene, fluorene, xanthene, acridine, perylene, terpenes and naphthenes.

The present hydrogenations may be carried out to produce totally saturated compounds corresponding to the starting material or in some instances compounds having reduced unsaturation from the starting material. Not uncommonly the hydrogenation results in the cession of the ring structure and some cracking.

The hydrogenations described herein are exothermic reactions. In the past the temperature has been controlled by quench at strategic points within a reactor by addition of cool hydrogen. The addition of the hydrogen also acted to maintain a molar excess of hydrogen within the reactor to prevent coking and other undesirable side reactions. It is believed that in the present reaction catalytic distillation is a benefit first, because the reaction is occurring concurrently with distillation, the initial reaction products and other stream components are removed from the reaction zone as quickly as possible reducing the likelihood of side reactions. Second, because all the components are boiling the temperature of reaction is controlled by the boiling point of the mixture at the system pressure. The heat of reaction simply creates more boil up, but no increase in temperature at a given pressure.

The present invention carries out the method in a catalyst packed column which can be appreciated to contain a vapor phase and some liquid phase as in any distillation. The distillation column reactor is operated at a pressure such that the reaction mixture is boiling in the bed of catalyst. The present process for hydrogenating benzene operates at overhead pressure of said distillation column reactor in the range between 101, preferably 343, to 929 kPa (0, preferably 35, to 120 psig) and temperatures in said distillation reaction bottoms zone in the range of 37° to 190°C (100 to 374°F) at the requisite hydrogen partial pressures.

With an aniline feed the present process operates at overhead pressure of said distillation column reactor in the range between 101, preferably 343, to 929 kPa (0, preferably 35, to 120 psig) and temperatures in said distillation reaction bottoms zone of 37° to 190°C (100° to 374°F) at the requisite hydrogen partial pressures.

The feed weight hourly space velocity (WHSV), which is herein understood to mean the unit weight of feed per hour entering the reaction distillation column per unit weight of catalyst in the catalytic distillation structures, may vary over a very wide range within the other condition perimeters, e.g. 0.1 to 35. Hydrogenation conditions used for other unsaturated cyclic and polycyclic compounds are similar to those for benzene, although some of the compounds may require higher temperatures to volatilize the materials of the process

In the current process the temperature is controlled by operating the reactor at a given pressure to allow partial vaporization of the reaction mixture. The exothermic heat of reaction is thus dissipated by the latent heat of vaporization of the mixture. The vaporized portion is taken as overheads and the condensible material condensed and returned to the column as reflux.

The downward flowing liquid causes additional condensation within the reactor as is normal in any distillation. The contact of the condensing liquid within the column provides excellent mass transfer for dissolving the hydrogen within the reaction liquid and concurrent transfer of the reaction mixture to the catalytic sites. It is thought that this condensing mode of operation results in the excellent conversion and selectivity of the instant process and allows operation at the lower hydrogen partial pressures and reactor temperatures noted. A further benefit that this reaction may gain from catalytic distillation is the washing effect that the internal reflux provides to the catalyst thereby reducing polymer build up and coking. Internal reflux may vary over the range of 0.2 to 20 L/D (wt. liquid just below the catalyst bed/wt. distillate) to give excellent results.

Hydrogen must be supplied in at least stoichiometric quantities. The preferred ratio is greater than 4:1 molar ratio of hydrogen to unsaturated cyclic compound, e.g. aniline. Ammonia may be provided to suppress the side reactions and promote the production of cyclohexyl amine. The molar ratio of ammonia to aniline is preferably at least 1:1. Hydrogen should be present in the molar ratio of about 4:1 of hydrogen to ammonia.

The aniline process can thus be considered to comprise:
(a) feeding a first stream containing aniline and a second stream containing hydrogen to a distillation column reactor;
(b) contacting the aniline and hydrogen at a hydrogen partial pressure less than 345 kPa (50 psia) in the presence of a hydrogenation catalyst prepared in the form of a catalytic distillation structure thereby reacting a portion of the aniline with a portion of the hydrogen to form a reaction mixture containing cyclohexyl amine, unreacted hydrogen and unreacted aniline;
(b) maintaining the pressure in the distillation column reactor such that the reaction mixture is at its boiling point;
(c) removing gaseous aniline, gaseous cyclohexyl amine and hydrogen as overheads from the distillation column reactor;
(d) condensing substantially all of the aniline and cyclohexyl amine removed as overheads from the distillation column reactor;
(e) returning a portion of the condensed aniline and cyclohexyl amine to the distillation column reactor as reflux; and
(f) withdrawing an overheads liquid product containing cyclohexyl amine from the distillation column.

The catalyst is prepared in the form of a catalytic distillation structure. More particularly the hydrogenation catalyst is generally a metal supported on an alumina carrier in the form of extrudates or spheres. The extrudates or spheres are placed in porous containers and suitably supported in the distillation column reactor to allow vapor flow through the bed, yet provide a sufficient surface area for catalytic contact.

Among the metals known to catalyze the hydrogenation reaction are platinum, rhenium, cobalt, molybdenum, nickel, tungsten and palladium. Generally, commercial forms of catalyst use supported oxides- of these metals. The oxide is reduced to the active form either prior to use with a reducing agent or during use by the hydrogen in the feed. These metals also catalyze other reactions, most notably dehydrogenation at elevated temperatures. Additionally they can promote the reaction of olefinic compounds with themselves or other olefins to produce dimers or oligomers as residence time is increased.

One preferred embodiment is for the production of cyclohexane from the hydrogenation of benzene. When cyclohexane is the product the benzene containing feed is characterized as preferably containing at least 5 wt% benzene up to 100 wt%. Other components are typically C₅, C₆ and C₇ hydrocarbons. Since other unsaturated compounds may be hydrogenated, the presence of these compounds is detrimental to the process when cyclohexane is the desired product. Preferably other unsaturated compounds should be limited to less than 30% of the feed. Cyclohexane is the preferred diluent, since it is the desired product. However, other inerts such as other alkanes are acceptable, such as C₅'s up to C₉'s.

The mole ratio of hydrogen to benzene fed to the distillation column reactor is preferably between 1.5:1 and 41:1.

As described the catalytic material employed in the hydrogenation process is in a form to serve as distillation packing. Broadly stated, the catalytic material is a component of a distillation system functioning as both a catalyst and distillation packing, i.e., a packing for a distillation column having both a distillation function and a catalytic function.

The reaction system can be described as heterogenous since the catalyst remains a distinct entity. Any suitable hydrogenation catalyst may be used, for example Group VIII metals of the Periodic Table of Elements as the principal catalytic component, alone or with promoters and modifiers such as palladium/gold, palladium/silver, cobalt/zirconium, nickel preferably deposited on a support such as alumina, fire brick, pumice, carbon, silica, resin or the like.

A preferred catalyst structure for the hydrogenation of benzene comprises at least one plurality of flexible, semi-rigid open mesh tubular elements filled with a particulate catalytic material (catalyst component) and sealed at both ends, intimately associated with and supported by a wire mesh screen coiled into a spiral having a longitudinal axis, said tubular element being arrayed at an angle to the longitudinal axis thereby forming a bale and is described in detail in U.S. Pat. No. 5,431,890.

The flexible, semi-rigid open mesh tubular element filed with a particulate catalytic material preferably has a fastener every 2.5 to 30 cm (1-12 inches) along the length of the tube to form a multiple link shaped catalytic distillation structure. The links formed by the fasteners may be evenly or irregularly spaced.

The bale shaped catalytic distillation structures are formed by placing at least one tubular element on top of the wire mesh screen, such as demister wire, in a diagonal array, such that when the wire mesh screen is rolled up, the rolled structure provides a new and improved catalytic distillation structure. Further embodiments include multiple stack arrangements of' alternating wire screen mesh and tubular elements that are rolled into a new bale shaped catalytic distillation structure. The tubular elements on alternating layers are preferably arrayed on the wire mesh screen in opposite directions such that their paths cross. Each tubular element will define a spiral within the bale.

The catalyst component may take several forms. In the case of particulate catalytic material, generally from 60 mm to about 1 mm down through powders, is enclosed in a porous container such as screen wire, or polymeric mesh. The material used to make the container must be inert to the reactants and conditions in the reaction system. The screen wire may be aluminum, steel, stainless steel, and the like. The polymer mesh may be nylon, teflon, or the like. The mesh or threads per cm (inch) of the material used to make the container is such that the catalyst is retained therein and will not pass through the openings in the material. Although the catalyst particles of about 0.15 mm size or powders may be used and particles up to about 6.35 mm (1/4 inch) diameter may be employed in the containers.

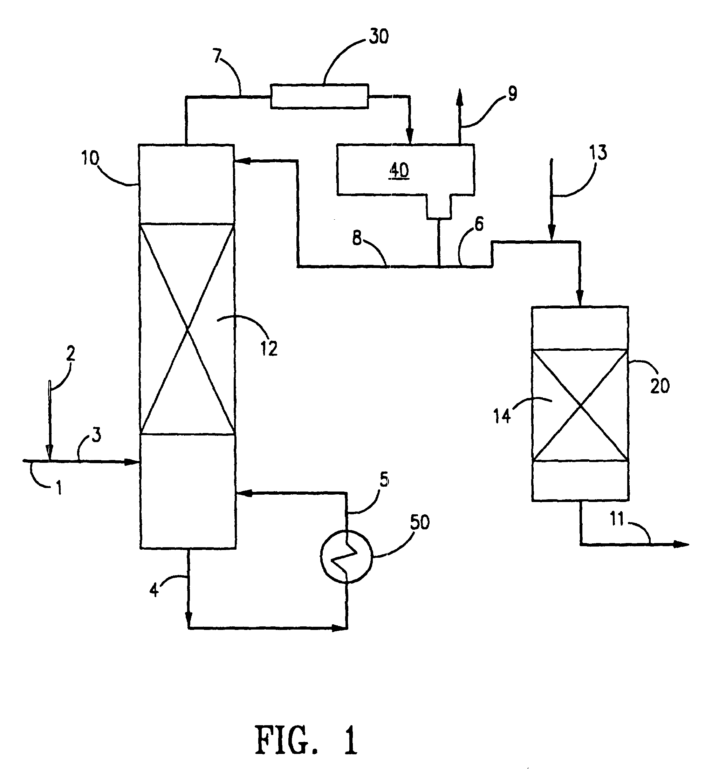

Referring now to the FIG. 1 there is shown a flow diagram of the benzene embodiment of the invention. Benzene is fed via line 1 and hydrogen via line 2 both being combined in line 3 which feeds the hydrogen and benzene below the catalytic distillation structure 12 contained in distillation column reactor 10. If desired the benzene feed may be diluted with cyclohexane. Heat necessary for start up and to balance the process is provided by circulating the bottoms stream 4 through reboiler 50 and return line 5. The benzene is boiled up into the bed where a portion reacts with hydrogen to form a reaction mixture containing the reaction product cyclohexane, unreacted benzene and unreacted hydrogen. The exothermic heat of reaction causes more boil up of the reaction mixture with the vaporized portion leaving the column as overheads via flow line 7. Unreacted hydrogen also exits with the overheads. The gaseous overheads containing benzene, cyclohexane and hydrogen are passed through condenser 30 where substantially all of the benzene and cyclohexane are condensed. The overheads stream is then passed to receiver/separator 40 where the gas which is mostly hydrogen is separated and the liquid collected. The gas is removed via line 9 for recycle or use later in the process.

A portion of the condensed liquid is returned to the distillation column as reflux where it provides additional cooling and condensing within the column. The bottoms, containing benzene and cyclohexane, are removed via flow line 4 with a portion being recirculated through reboiler 50 and flow line 5. There is no bottoms product stream taken. The overheads liquid product stream is finally passed via flow line 6 to single pass fixed bed reactor 20 containing a fixed bed of hydrogenation catalyst 14 where substantially all of the unreacted benzene is hydrogenated to cyclohexane. Hydrogen is provided to second reactor 20 via flow line 13 which may conveniently be taken from the vent 9 of overhead receiver 40 if desired.

The present process allows for the use of much lower hydrogen partial pressures and somewhat lower temperatures than normal processes.

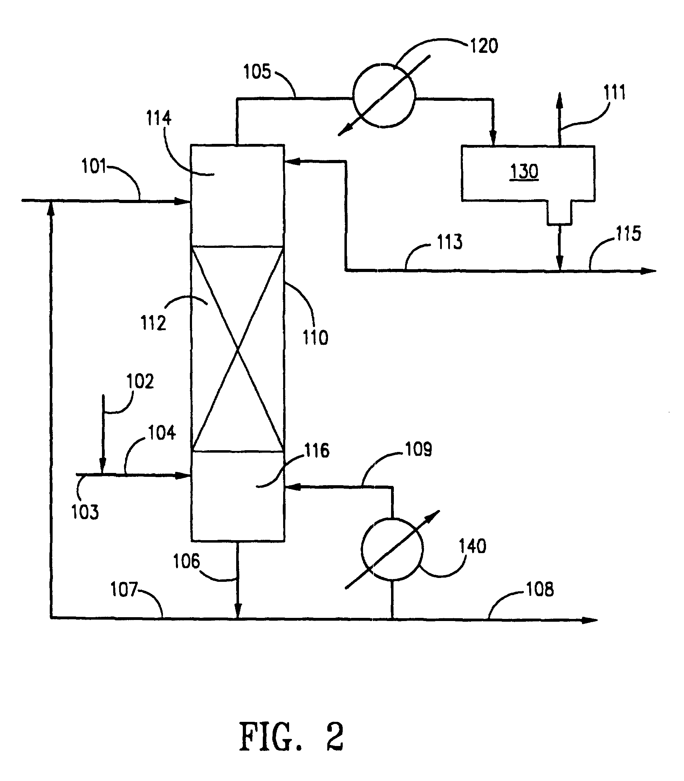

Referring now to the FIG. 2 there is shown a flow diagram of one embodiment of the invention. Aniline is fed via line 101 to the distillation column reactor 110 at a point above the catalyst bed 112 containing the catalytic distillation structure. Hydrogen is fed via flow line 102 and ammonia via flow line 103 which are combined in flow line 104 and fed below the bed 112. Overheads containing the cyclohexyl amine and unreacted aniline and hydrogen are taken via flow line 105 and passed through partial condenser 120 wherein the condensible materials are condensed. The overheads are then collected in receiver/separator 130 wherein the hydrogen and other uncondensed vapors, e.g., ammonia, are separated and removed via flow line 111 for recycle (not shown) if desired to the hydrogen feed or at a point below the catalyst bed. Product is taken via flow line 115 and a portion of the condensed overheads is returned to the distillation column reactor 110 as reflux via flow line 113.

Bottoms are taken via flow line 106 and a portion are passed through reboiler 140 to balance heat for the column 110. The bottoms contain heavy by-products which include cyclohexyl phenyl amine and dicyclohexyl amine. A portion of the bottoms may be recycled to the feed via flow line 107 for conversion to cyclohexyl amine. A bottoms draw via flow line 108 is provided to prevent build up of the heavies.

The distillation column reactor is provided with rectifying section 114 to separate unreacted aniline from the product and stripping section 116 to insure that no aniline or product is removed as bottoms.

In Examples 1-3 a 2.54 cm (1 inch) diameter distillation column reactor was used. The catalyst structure as described above was placed in the top 4m (13 feet) of the reactor. The bottom 2 m (7 feet) were filled with inert distillation packing. The overhead pressure was set as desired and the reboiler was charged with cyclohexane and heat added. When the desired top to bottom temperature differential was obtained the liquid feed rate was established and hydrogen flow started. After a level was noted in the overhead receiver cyclohexane flow was stopped and the unit operated at total reflux for two hours before the benzene/cyclohexane feed was started. Overheads liquids product draw was set to balance the column.

### EXAMPLE 1

400 grams of 1.6 mm (1/16 inch) spherical alumina supported nickel (54 wt.% Ni) catalyst were loaded into the tubular elements and wound into a bale as previously described and placed into the distillation column reactor. Conditions and results are shown in Table I below.

**TABLE I**

| | | | |
|---|---|---|---|
| Time on stream, hrs | 51 | 134.8 | 219 |
| Pressure, kPa (psig) | 515 (60) | 343 (35) | 343 (35) |
| Bottoms Temp. °C (°F) | 176 (349) | 100 (212) | 104 (219) |
| Internal Reflux Ratio | 23.3 | 2.5 | 2.5 |
| Feed Rate, kg/hr (lbs/hr) liq. | 0.45 (1) | 0.45 (1) | 0.45 (1) |
| H2 Rate m³/hr (scfh), gas | 0.21 (7.5) | 0.21 (7.5) | 0.21 (7.5) |
| H2/Bz mole ratio | 4.2 | 4.2 | 4.2 |
| Benzene in feed, wt% | 36 | 36 | 36 |
| Pressure Drop kPa (psi) | 24.68 (3.58) | 24.82 (3.60) | 20.68 (3.00) |
| H2 pp, kPa (psia) overhead analysis, wt% | 32.68 (4.74) | 151.14 (21.92) | 151.14 (21.92) |
| Cyclohexane | 77.68 | 82.68 | 91.50 |
| Benzene | 21.75 | 17.22 | 8.50 |

### EXAMPLE 2

280 grams of alumina supported platinum/palladium (.3 wt.% Pt, .5 wt.% Pd) catalyst were loaded into the tubular elements and wound into a bale as described above and placed into the distillation column reactor. Conditions and results are shown in Table II below.

**TABLE II**

| | | |
|---|---|---|
| Time on stream, hrs | 148 | 346 |
| Pressure, kPa (psig) | 791 (100) | 929 (120) |
| Bottoms Temp. °C (°F) | 180 (356) | 190 (374) |
| Internal Reflux Ratio | 23.2 | 23.1 |
| Feed Rate, kg/hr (lbs/hr) liq. | 0.45 (1) | 0.45 (1) |
| H2 Rate m³/hr (scfh), gas | 0.28 (10.0) | 0.28 (10.0) |
| | | |
| Benzene in feed, wt% | 36 | 5 |
| H2/Bz mole ratio | 5.6 | 40.6 |
| Pressure Drop kPa (psi) | 26.55 (3.85) | 26.20 (3.80) |
| | | |
| H2 pp, kPa (psia) 32.68 | 67.02 (9.72) | 79.43 (11.52) |
| overhead analysis, wt% | | |
| Cyclohexane | 76.56 | 99.88 |
| Benzene | 23.4 | 0.067 |

### EXAMPLE 3

400 grams of 4.8 mm (3/16 inch) inch alumina supported nickel (54 wt% Ni) tablets were loaded into the tubular elements and wound into a bale as described above and placed into the distillation column reactor. Conditions and results are shown in Table III below.

**TABLE III**

| | | | |
|---|---|---|---|
| Time on stream, hrs | 43 | 331 | 493 |
| Pressure, kPa (psig) | 515 (60) | 791 (100) | 791 (100) |
| Bottoms Temp. °C (°F) | 158 (316) | 179 (355) | 179 (355) |
| Internal Reflux Ratio | 23.4 | 29.7 | 29.7 |
| Feed Rate, kg/hr (lbs/hr) liq. | 0.91 (2.0) | 0.91 (2.0) | 0.91 (2.0) |
| H2 Rate m³/hr (scfh), gas | 0.21 (7.5) | 0.21 (7.5) | 0.28 (10.0) |
| Benzene in feed, wt% | 5.9 | 13.7 | 4.9 |
| H2/Bz mole ratio | 25.9 | 11.1 | 41.5 |
| Pressure Drop kPa (psi) | 26.27 (3.81) | 29.10 (4.22) | 27.58 (4.00) |
| H2 pp, kPa (psia) | 16.34 (2.37) | 20.20 (2.93) | 27.01 (3.92) |
| overhead analysis, wt% | | | |
| Cyclohexane | 96.57 | 90.06 | 98.91 |
| Benzene | 3.41 | 9.93 | 1.09 |

The preferred embodiment has been shown to be for the hydrogenation of benzene. However, the invention also covers the hydrogenation of any stream containing cyclic and polycyclic unsaturates as a T-90+ gasoline bottoms. Such unsaturates include aromatics, polynuclear aromatics, and cyclic alkenes such as naphthenes.

In the following examples a 7.6 meter (twenty five foot) tall 2.54 cm (one inch) diameter distillation column reactor was used. The catalyst used was Calsicat E-475 SR, 56% nickel on alumina in the form of 4.8 mm (3/16 inch) spheres. The catalyst was packaged as 15 cm (six inch) long by 2 cm (0.75) inch diameter "sausages" as described above wrapped with 0.149 mm sieve opening mesh (100 mesh) stainless steel screen.

### EXAMPLE 4

In this example 0.30 kg (0.66 pound) of the Calsicat E-475 prepared as described above was loaded into the middle 3 meters (10 feet) of the distillation column reactor. The top and bottom 2.3 meters (7.5 feet) were packed with ceramic saddles. Conditions and results are summarized in Table IV below. The data show that pressure (and consequently temperature in the catalyst zone) had the greatest leverage on productivity ranging from -0.068 kg/kg (-0.15 lbs/lb) catalyst at 446 kPa (50 psig) to 0.18 kg/kg (-0.4 lbs/lb) at 1136 kPa (150 psig). Selectivity to cyclohexyl amine was in the 65-75% range with the main by-products being cyclohexyl phenyl amine and dicyclohexyl amine.

**TABLE IV**

| | | | | |
|---|---|---|---|---|
| Time On Stream, Hrs. | 100 | 160 | 210 | 260 |
| Pressure kPa, (psig) | 446(50) | 791(100) | 1136(150) | 446(50) |
| Temperature, °C (°F) | | | | |
| Overhead | 138(280) | 149(300) | 138(280) | 177(350) |
| Cat. Bed | 177(350) | 193(380) | 204(400) | 204(400) |
| Bottoms | 260(500) | 304(580) | 327(620) | 277(530) |
| Aniline Feed, kg/hr (lbs/hr) | 0.18(0.4) | 0.18(0.4) | 0.27(0.6) | 0.23(0.5) |
| Overhead, kg/hr (lbs/hr) | 0.14(0.3) | 0.14(0.3) | 0.23(0.50) | 0.14(0.3) |
| Cyclohexyl amine, wt% | 15 | 25 | 45 | 8 |
| Bottoms, kg/hr (lbs/hr) | 0.045(0.1) | 0.045(0.1) | 0.09(0.2) | 0.045(0.1) |
| Hydrogen Rate, m³/hr (SCFH) | 0.43(15) | 0.43(15) | 0.43(15) | 0.43(15) |
| Ammonia, kg/hr (lbs/hr) | 0.045(0.1) | 0.045(0.1) | 0.09(0.2) | 0.045(0.1) |
| Cyclohexyl Amine | | | | |
| Selectivity, wt% | 66 | 71 | 77 | 41 |
| Total Amines | | | | |
| Productivity, kg/hr/0.45kg (lbs/hr/lb) | 0.05(0.11) | 0.10(0.23) | 0.18(0.4) | 0.054(0.12) |

### EXAMPLE 5

In this example 0.45 kg (1.0 pound) of the Calsicat E-475 prepared as described above was loaded into the bottom 4.6 meters (15 feet) of the distillation column reactor. The top 3 meters (10 feet) were packed with ceramic saddles. Conditions and results are summarized in Table V below. During part of the run (from about 250 hours onward) the heavy bottoms product containing the cyclohexyl phenyl amine and dicyclohexyl amine were recycled at a rate of 20 wt.% of the fresh feed.

**TABLE V**

| | | | |
|---|---|---|---|
| Time On Stream, Hrs. | 100 | 200 | 300 |
| Pressure kPa, (psig) | 446(50) | 446(50) | 446(50) |
| Aniline Feed, kg/hr (lbs/hr) | 0.23(0.5) | 0.36(0.8) | 0.36(0.8) |
| Overhead, kg/hr (lbs/hr) Cyclohexyl amine, wt% | 0.18(0.4) | 0.32(0.7) | 0.32(0.7) |
| Bottoms, kg/hr (lbs/hr) | 0.045(0.1) | 0.045(0.1) | 0.045(0.1) |
| Hydrogen Rate, m³/hr (SCFH) | 0.43(15) | 0.57(20) | 0.57(20) |
| Ammonia, kg/hr (lbs/hr) | 0.045(0.1) | 0.045(0.1) | 0.045(0.1) |
| Cyclohexyl Amine | | | |
| Selectivity, wt% | 66 | 72 | 71 |
| Total Amines | | | |
| Productivity, kg/hr/0.45kg (lbs/hr/lb) | 0.14(0.3) | 0.18(0.4) | 0.16(0.35) |

## Claims

1. A process for the hydrogenation of unsaturated cyclic compounds comprising the steps of:
(a) feeding a first stream containing unsaturated cyclic compounds and a second stream containing hydrogen to a distillation column reactor;
(b) contacting the unsaturated cyclic compounds and hydrogen at a temperature in the range of 37° to 190°C (100° to 374°F), a hydrogen partial pressure of less than 345 kPa (50 psia), and an overhead pressure in the range of 101 to 929 kPa (0 to 120 psig) in the presence of a bed of hydrogenation catalyst prepared in the form of a catalytic distillation structure thereby reacting a portion of the unsaturated cyclic compounds with a portion of the hydrogen to form a reaction mixture containing saturated cyclic compounds, unreacted hydrogen and unreacted unsaturated cyclic compounds;
(c) maintaining the pressure in the distillation column reactor (i) to contain a vapor phase and some liquid phase and provide a continual reflux such that the reaction mixture is at its boiling point and boiling in the bed of catalyst and (ii) condensing a portion of the vapors in the reaction system whereby a portion of the aromatics and other unsaturated cyclic and polycyclic compounds is always condensing on the catalyst structure;
(d) removing gaseous unsaturated cyclic compounds, gaseous saturated cyclic compounds and hydrogen as overheads from the distillation column reactor;
(e) condensing substantially all of the unsaturated cyclic compounds and saturated cyclic compounds removed as overheads from the distillation column reactor;
(f) returning a portion of the condensed unsaturated cyclic compounds and saturated cyclic compounds to the distillation column reactor as reflux; and
(g) withdrawing a liquid product containing saturated cyclic compounds and unreacted unsaturated cyclic compounds from the condensed overheads.

2. The process according to claim 1, wherein the overhead pressure of the distillation column reactor is from 343 to 929 kPa (from 35 to 120 psig).

3. The process according to claim 1, wherein the hydrogen partial pressure is from 14 to 172 kPa (from 2 to 25 psia).

4. The process according to claim 1, wherein said catalytic distillation structure comprises a first plurality of flexible, semi-rigid open mesh tubular elements filled with a particulate hydrogenation catalytic material, sealed at both ends, intimately associated with and supported by a wire mesh screen coiled into a spiral having a longitudinal axis, said tubular elements being arrayed at an angle to the longitudinal axis.

5. The process according to any claims 1 to 4, wherein said unsaturated cyclic compound comprises aniline and the saturated cyclic compound comprises cyclohexyl amine.

6. The process according to claim 5, wherein the vapor product after condensing the overheads is recycled to the distillation reactor with the hydrogen feed or at another place below the catalyst bed.

7. The process according to claim 5 or 6, further comprising the step of feeding a third stream containing ammonia to said distillation column reactor.

8. The process according to any of claims 5 to 7, wherein cyclohexyl phenyl amine and dicyclohexyl amine formed as by-products are removed from said distillation column reactor as bottoms, a portion of said bottoms is recycled as feed to said distillation column reactor along with said aniline and a portion of the cyclohexyl phenyl amine and dicyclohexyl amine is converted to cyclohexyl amine.

9. The process according to any of claims 1 to 4, wherein said unsaturated cyclic compound comprises benzene and the saturated cyclic compound comprises cyclohexane.

10. The process according to claim 9, wherein the first stream comprises 5-36 weight percent benzene, and/or the remainder of said first stream comprises cyclohexane.

11. The process according to claim 9 or 10, comprising the step of passing overheads liquid product containing cyclohexane and unreacted benzene along with hydrogen to a single pass fixed bed reactor containing a hydrogenation catalyst to react substantially all of the unreacted benzene with hydrogen to produce additional cyclohexane.

12. The process according to any of claims 9-11, wherein the bottoms temperature of the distillation column reactor is from 100° to 190°C (from 212 to 374°F).

## Patentansprüche

1. Verfahren für die Hydrierung von ungesättigten cyclischen Verbindungen, umfassend die Schritte:
(a) Zuführen eines ungesättigte cyclische Verbindungen enthaltenden ersten Stroms und eines Wasserstoff enthaltenden zweiten Stroms zu einem Destillationssäulenreaktor;
(b) Kontaktieren der ungesättigten cyclischen Verbindungen und des Wasserstoffes bei einer Temperatur in Bereich von 37° bis 190°C (100° bis 374°F), einem Wasserstoffpartialdruck von weniger als 345 kPa (50 psia) absolutem Druck und einem Überkopfdruck im Bereich von 101 bis 929 kPa (Null bis 120 psig) Überdruck in Gegenwart eines Bettes von Hydrierungskatalysator, hergestellt in Form einer katalytischen Destillationsstruktur, dadurch Umsetzen eines Teils der ungesättigten cyclischen Verbindungen mit einem Teil des Wasserstoffs, um ein Reaktionsgemisch zu erzeugen, das gesättigte cyclische Verbindungen enthält, nichtumgesetzten Wasserstoff sowie nichtumgesetzte ungesättigte cyclische Verbindungen;
(c) Aufrechterhalten des Druckes in dem Destillationssäulenreaktor (i), so dass eine Dampfphase und etwas Flüssigphase enthalten ist und ein kontinuierlicher Rückfluss gewährt wird derart, dass sich das Reaktionsgemisch bei seinem Siedepunkt befindet; und Siedenlassen in dem Bett des Katalysators, sowie (ii) Kondensieren eines Teils des Dampfes in dem Reaktionssystem, wodurch ein Teil der Aromaten und anderer ungesättigter cyclischer und polycyclischer Verbindungen ständig an der Katalysatorstruktur konensiert wird;
(d) Entfernen der gasförmigen, ungesättigten cyclischen Verbindungen; der gasförmigen, gesättigten cyclischen Verbindungen und des Wasserstoffs als Überkopfprodukte aus dem Destillationssäulenreaktor;
(e) Kondensieren weitgehend aller ungesättigten cyclischen Verbindungen und gesättigten cyclischen Verbindungen, die als Überkopfprodukte aus dem Destillationssäulenreaktor entfernt wurden;
(f) Rückführen eines Teils der kondensierten, ungesättigten cyclischen Verbindungen und gesättigten cyclischen Verbindungen in den Destillationssäulenreaktor als Rückfluss; und
(g) Abziehen eines gesättigte cyclische Verbindungen und nichtumgesetzte, ungesättigte cyclische Verbindungen enthaltenden flüssigen Produkts aus den kondensierten Überkopfprodukten.

2. Verfahren nach Anspruch 1, bei welchem der Überkopfdruck des Destillationssäulenreaktors 343 bis 172 kPa (35 bis 120 psig) Überdruck beträgt.

3. Verfahren nach Anspruch 1, bei welchem der Wasserstoffpartialdruck 14 bis 172 kPa (2 bis 25 psia) Absolutdruck beträgt.

4. Verfahren nach Anspruch 1, bei welchem die katalytische Destillationsstruktur eine erste Vielzahl von flexiblen, halbsteifen offenmaschigen, röhrenförmigen Elementen aufweist, gefüllt mit einem partikulären Material für die katalytische Hydrierung an beiden Enden gasdicht verschlossen, eng miteinander vereinigt und zusammengehalten durch ein Drahtgewebesieb, das zu einer Spirale gewickelt ist, die eine Längsachse hat, wobei die röhrenförmigen Elemente in einem Winkel zur Längsachse angeordnet sind.

5. Verfahren nach einem der vorgenannten Ansprüche 1 bis 4, bei welchem die ungesättigte cyclische Verbindung Anilin umfasst und die gesättigte cyclische Verbindung Cyclohexylamin umfasst.

6. Verfahren nach Anspruch 5, bei welchem das Dampfprodukt nach dem Kondensieren der Überkopfprodukte in den Destillationsreaktor mit dem Wasserstoff-Aufgabegut zurückgeführt wird oder zu einer anderen Stelle unterhalb des Katalysatorbetts.

7. Verfahren nach Anspruch 5 oder 6, ferner umfassend die Schritte des Zuführens eines Ammoniak enthaltenden dritten Stroms in den Destillationssäulenreaktor.

8. Verfahren nach einem der vorgenannten Ansprüche 5 bis 7, bei welchem Cyclohexylphenylamin und Dicyclohexylamin, die als Nebenprodukte erzeugt werden, aus dem Destillationssäulenreaktor als Bodenprodukte enffernt werden, und ein Teil der Bodenprodukte als Aufgabegut in den Destillationssäulenreaktor zusammen mit Anilin zurückgeführt wird und Cyclohexylphenylamin und Dicyclohexylamin zu Cyclohexylamin umgesetzt werden.

9. Verfahren nach einem der vorgenannten Ansprüche 1 bis 4, bei welchem die ungesättigte cyclische Verbindung Benzol umfasst und die gesättigte cyclische Verbindung Cyclohexan umfasst.

10. Verfahren nach Anspruch 9, bei welchem der erste Strom 5% bis 36% Benzol aufweist und/oder der Rest des ersten Stroms Cyclohexan aufweist.

11. Verfahren nach Anspruch 9 oder 10, umfassend den Schritt des Durchleitens des Überkopfprodukte enthaltenden flüssigen Produkts, das Cyclohexan und nichtumgesetztes Benzol enthält, zusammen mit Wasserstoff zu einem Einzeldurchlauf-Festbettreaktor, enthaltend einen Wasserstoff-Katalysator, um weitgehend das gesamte nichtumgesetzte Benzol mit Wasserstoff umzusetzen und um zusätzliches Cyclohexan zu erzeugen.

12. Verfahren nach einem der vorgenannten Ansprüche 9 bis 11, bei welchem die Temperatur der Bodenprodukte des Destillationssäulenreaktors 100° bis 190°C (212° bis 374°F) beträgt.

## Revendications

1. Procédé pour l'hydrogénation de composés cycliques insaturés comprenant les étapes de :
(a) alimenter un premier courant contenant des composés cycliques insaturés et un second courant contenant de l'hydrogène vers un réacteur à colonne à distillation ;
(b) mettre en contact les composés cycliques insaturés et l'hydrogène à une température dans le domaine de 37° à 190°C (100° à 374°F), à une pression partielle en hydrogène de moins de 345 kPa (50 psia), et une pression en tête dans le domaine de 101 à 929 kPa (0 à 120 psig) en présence d'un lit de catalyseur d'hydrogénation préparé sous la forme d'une structure de distillation catalytique, en mettant en réaction une partie des composés cycliques insaturés avec un partie de l'hydrogène pour produire un mélange réactionnel contenant des composés cycliques saturés, de l'hydrogène n'ayant pas agi et des composés cycliques insaturés n'ayant pas réagi ;
(c) maintenir la pression dans le réacteur à colonne à distillation (i) pour contenir une phase vapeur et quelques phases liquides et apporter un reflux continu de sorte que le mélange réactionnel soit à son point d'ébullition et qu'il bout dans le lit de catalyseur et (ii) condenser une partie des vapeurs dans le système réactionnel dans lequel une partie des aromatiques et des autres composés polycycliques et cycliques insaturés sont toujours condensés sur la structure catalytique ;
(d) éliminer les composés cycliques insaturés gazeux, les composés cycliques saturés gazeux et l'hydrogène en tête du réacteur à colonne à distillation ;
(e) condenser essentiellement tous les composés cycliques insaturés et les composés cycliques saturés éliminés en tête du réacteur à colonne à distillation ;
(f) renvoyer une partie des composés cycliques insaturés condensés et des composés cycliques saturés vers le réacteur à colonne à distillation sous la forme d'un reflux ; et
(g) retirer un produit liquide contenant les composés cycliques saturés et les composés cycliques insaturés n'ayant pas réagi de la tête condensée.

2. Procédé selon la revendication 1, **caractérisé en ce que** la pression en tête du réacteur à colonne à distillation est de 343 à 949 kPa (de 35 à 120 psig).

3. Procédé selon la revendication 1, **caractérisé en ce que** la pression partielle en hydrogène est de 14 à 172 kPa (de 2 à 25 psia).

4. Procédé selon la revendication 1, **caractérisé en ce que** ladite structure de distillation catalytique comprend une première pluralité d'éléments tubulaires, tressés, ouverts, semi-rigides, flexibles, remplis avec un matériau catalytique d'hydrogénation particulaire, scellés à leurs deux extrémités, intimement associés avec, et supportés par, un écran à mailles en fils enroulés en spirale ayant un axe longitudinal, lesdits éléments tubulaires étant arrangés avec un angle par rapport à l'axe longitudinal.

5. Procédé selon Tune quelconque des revendications 1 à 4, **caractérisé en ce que** ledit composé cyclique insaturé comprend de l'aniline et **en ce que** le composé cyclique saturé comprend de la cyclohexylamine.

6. Procédé selon la revendication 5, **caractérisé en ce que** le produit gazeux, après que la tête soit condensée, est recyclé vers le réacteur à distillation avec l'alimentation d'hydrogène ou à un autre endroit en-dessous du lit de catalyseur.

7. Procédé selon Tune quelconque des revendications 5 ou 6, **caractérisé en ce qu'**il comprend en outre l'étape d'alimenter ledit réacteur à colonne à distillation avec un troisième courant contenant de l'ammoniac.

8. Procédé selon Tune quelconque des revendications 5 à 7, **caractérisé en ce que** la cyclohexylphénylamine et la dicyclohexylamine formées comme sous-produits sont éliminées dudit réacteur à colonne à distillation sous la forme de pied, une portion dudit pied étant recyclée sous la forme d'alimentation dudit réacteur à colonne à distillation avec ladite aniline et une partie de la cyclohexylphénylamine et de la dicyclohexylamine sont converties en cyclohexylamine.

9. Procédé selon l'une quelconque des revendications 1 à 4, **caractérisé en ce que** ledit composé cyclique insaturé comprend du benzène et **en ce que** le composé cyclique saturé comprend du cyclohexane.

10. Procédé selon la revendication 9, **caractérisé en ce que** le premier courant comprend de 5 à 36% en poids de benzène, et/ou le reste dudit premier courant comprend du cyclohexane.

11. Procédé selon la revendication 9 ou 10, **caractérisé en ce qu'**il comprend l'étape consistant à faire passer des produits liquides de tête contenant du cyclohexane et du benzène n'ayant pas réagi avec de l'hydrogène dans un réacteur à lit fixe à une seule passe, contenant un catalyseur d'hydrogénation, pour faire réagir essentiellement tout le benzène n'ayant pas réagi avec l'hydrogène pour produire du cyclohexane supplémentaire.

12. Procédé selon l'une quelconque des revendications 9 à 11, **caractérisé en ce que** la température de pied du réacteur à colonne à distillation est de 100° à 190°C (de 212 à 374°F).
